# EUROPEAN PATENT APPLICATION

(11) **EP 1 990 638 A1**
(43) Date of publication of application: **12.11.2008**
(21) Application number: 07108038.6
(22) Date of filing: 11.05.2007
(51) Int. Cl.: G01N 33/543

(54) **Flow-through biosensor**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention provides a device for detecting an analyte in a sample comprising a capture probe module, wherein that capture probe module comprises (i) a segment of a flow channel comprising microchannels and (ii) a plurality of elements comprising at least one capture probe or alternatively being capable of immobilizing at last one capture probe for detecting said analyte, wherein said elements are arranged in said flow channel in such an order that adjacent elements form part of said microchannels.

## Description

### FIELD OF THE INVENTION

The present invention relates to a flow-through diagnostic device including a biosensor for detecting an analyte in a fluid.

### BACKGROUND OF THE INVENTION

Devices for molecular diagnostics are generally built around substrates that contain patterns of biosensors for detecting the presence of specific analytes in a sample. The biosensors generally comprise receptor molecules that are capable of binding with the biomolecular analytes to be detected, such as DNA, RNA, proteins, cells or drugs present within the sample. For example, for detecting whether an antigen is present in the sample, antibody binding may be used. For detecting the presence of DNA, protein interactions may be applied or a piece of a complimentary single-strand DNA.

Various detection methods have been developed to sense whether the specific binding has occurred. Commonly, an optical detection method is used that is based on, for example, fluorescence, refractive index changes or spectral changes. Also localized changes in magnetic or dielectric behaviour are being used. In a particular example of an optical biosensor, a membrane substrate is provided with an array of capture targets of different receptor molecules, for example, applied via inkjet printing and/or contact printing. The receptor molecules are brought into contact with the sample containing the analyte via a direct contact in a well plate or in a micro-channel using a so-call flow-over principle. In order to enhance the sensitivity of the detection device and for reducing the assay time needed to diagnose the sample, a flow-through principle may alternatively be used.

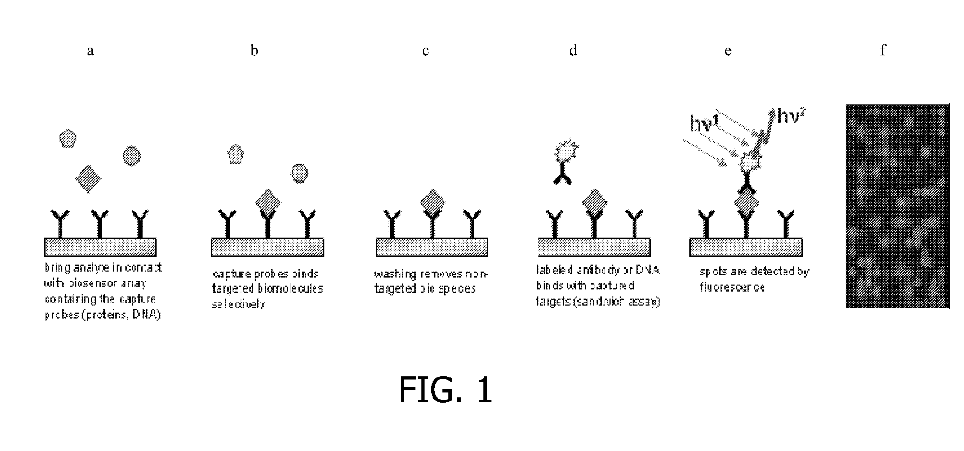

In order to be detected, the biomolecules of interest, that are the analytes, have to bind specifically to the corresponding capture probes. This step is often referred to as either hybridization, in case of a DNA analyte or incubation, in case of a protein analyte. For determining whether the specific capturing took place, fluorescent labeled single strand DNA (ssDNA) fragments or antibodies may be used which will bind to the capture probe: analyte complex. The presence of the specific biomolecules can then be detected by means of an optical detection device, such as a CCD camera if the fluorescent probes are excited to emit light. In Figure 1, this basic principle of a microarray is shown when using a sandwich immunoassay. Figure 1(f) shows an example of a fluorescence pattern obtained from such a microarray.

As indicated above, the analyte of interest can be brought to the capture site of the diagnostic device either by flow-through or by flow-over. These two types of sample introduction give rise to two types of microarrays which mainly differ in the flow direction of the analyte towards the detection sites. In a flow-over device, the analyte flows parallel to a solid substrate. The substrate comprises an array of nanowells in which the capture molecules are located. The flow-over concept exhibits a low-pressure drop of the analyte flowing through the device. It is further possible to arrange the different biosensors in spots of a small size. A microarray of the flow-over type may be manufactured in such a way that the different spots are in series and the analyte may hit every capture probe. In practice, this often is not the case as the flow in a microfluidic device normally is laminar so that the transport of the target molecules, that is, the analytes, to the capture sites should occur by diffusion. This results in long assay times if the channel depth is large. In addition, a flow-over device has a relatively low sensitivity due to the limitation of the number of capture probes per projected area.

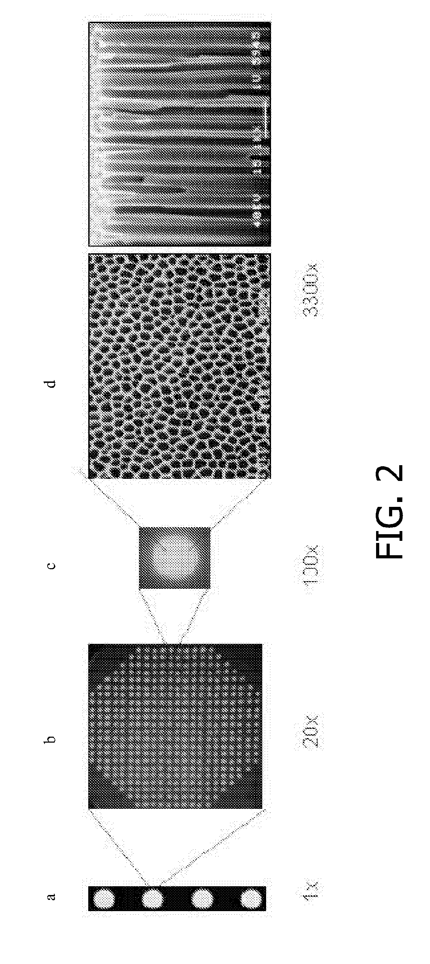

Some of these drawbacks of flow-over devices are overcome in a flow-through device in which the analyte flows perpendicularly through a microporous substrate comprising immobilized capture molecules. The high specific surface area of the microporous substrate causes a large number of bonded probe molecules on the detection spots, resulting in a high sensitivity for the detection of the respective biomolecular analytes. A drawback of flow-through devices is, however, that the capture areas are placed in parallel and that therefore a specific targeted area misses many analyte molecules as they pass outside the targeted area. In order to solve this problem, the sample is usually pumped through the spotted membrane several times. In Figure 2, an example of a commercially available membrane for use in a flow-through device is shown. The membrane shown in Figure 2 is available from Pamgene and is based on etched pores in an aluminum oxide membrane. Figure 2 shows the Pamgene membrane in different enlargements. A strip showing four areas that are printed with a microarray is shown in Figure 2(a). That the enlargement shown in Figure 2(b) of one of the areas, the array of spots comprising the various capture probes can be identified, an enlargement of one of these spots being shown in Figure 2(c). In Figure 2(d) the nanoporous membrane is shown in a top view and in a cross-sectional view. The inner surfaces of the membrane pores are coupled to the capture probes.

Both, the flow-over and the flow-through diagnostic devices thus exhibit their own merits and demerits. The flow-over device, built-in in a microchannel, requires only small sample volumes. However, the capture probe density is limited which leads to relatively low sensitivity and also to a low capture probability of the target molecules in a fast assay. On the other hand, the flow-through device provides a higher signal-to-noise ratio, but larger sample volumes are needed. Furthermore, the sample has to be pumped through the membrane to enhance the sensitivity for low concentrations of analytes.

WO 2006/062312 describes a biosensor for an on-the-spot analysis where commercial membranes are incorporated into microfluidic channels engraved on the surface of a plastic chip. WO 2007/012975 describes a hybrid device that combines a microfluidic component and a porous component.

### SUMMARY OF THE INVENTION

There is a need for an improved detection device for detecting analytes in a fluid. It is thus an object of the present invention to provide a diagnostic device which overcomes at least some of the limitations of the known devices. In particular, the diagnostic device of the present invention should provide a high signal-to-noise ratio while only requiring small sample volumes and a high surface/volume area. The device should further provide for rapid measuring protocols and should be easy to handle and to integrate.

In order to achieve these goals, the present invention provides a device for detecting an analyte in a sample, comprising a capture probe module, wherein that capture probe module comprises
a segment of a flow channel comprising microchannels and
a plurality of elements
comprising at least one capture probe or alternatively being capable of immobilizing at least one capture probe for detecting said analyte, wherein said elements are arranged in said flow channel in such an order that adjacent elements form part of said microchannels.

If elements do not comprise the capture probes said elements may at least be capable to immobilize capture probes. Different ways of immobilizing capture probes on the elements are described below.

The flow channel is preferably a microfluidic channel. Said channel has a width of less than 1000 µm, preferably a width of less than 300 µm and most preferably less than 150 µm. Said channel has a length of preferably less than 1000 µm, more preferably less than 500 µm, most preferably less than 200 µm but at least more than 1 µm and preferably more than 10 µm.

In a preferred embodiment the channels may be closed from their top side in order to avoid transport of analyte along unwanted voids left between the capture probe modules by adhering a cover plate covered with soft compliant material facing the channels and the capture probe modules. In a preferred embodiment the compliant material loosely crosslinked polydimethyl-siloxane.

The flow channel may comprise more than one of said capture probe modules, preferably a plurality of such capture probe modules, each containing probe molecules for another biomolecular analyte. In the case of a plurality of capture probe modules also some capture probe modules may be provided with the same capture probe molecules, e.g. to decrease the number of faults by multiple detection or to get information of the analyte concentration in the sample by comparison of the detection signal, e.g. fluorescence intensity. Also some capture probe modules may also be provided with signal providing probes, e.g. fluorescent probes, to mark and identify the positions of the other capture probe modules and also to determine the recovery.

The membranes may comprise elements which may have an elongate, plate-like form and may be arranged in parallel with the channel and preferably in parallel to each other separated by a predetermined distance. The elements may also have the form of pillars extending perpendicularly through the flow channel. That is, the elements are arranged in the flow channel to at least partly extend into the channel. Rather than using a flow-through membrane consisting of parallel plates also other shapes and arrangements can be chosen, such as interrupted plates, oblique plates, small pillars, horizontal pores and zig zag plates. Alternatively, plates with horizontal pores can be positioned perpendicular to the channel.

The lamella or other shapes which forms the basic structure is called herein an element. An assembly of elements forms the "integrated membrane" according to the present invention. It is not meant that the elements consist necessarily of membrane-like material. The definition of "integrated membrane" shall not limit this term to a certain material. The arrangement of the elements leads to a membrane-like structure or "integrated membrane". So, while passing through the channel the sample passes the "integrated membranes" thus coming in contact with the "element" coated with the capture probes.

Preferably the elements, which together form the membrane, should have a thickness of about 5 µm, more preferably of about 2 µm. In a preferred embodiment the elements are separated by about 2 µm in the capture probe module.

The membranes have a length of less than 1000 µm, preferably a length of less than 300 µm and most preferably less than 150 µm. The width of said membranes is less than 1000 µm, preferably a less than 300 µm and most preferably less than 150 µm. The width of said membranes may be larger than the width of the flow channel by creating a local expansion of the channel to fit said membrane or may be smaller by creating locally a contraction. The spacing between the membranes should be at least more than 1 micron, preferably more than 10 micron and most preferably more than 40 micron.

The capture probe module comprising the capture probes printed on the membrane and being capable of immobilizing the capture probes is designed such that they have a large internal surface area, providing large capture probe density on a small projected surface area, providing high intensity and high resolution. Several capture probe modules may be arranged in series.

As an alternative embodiment of the device one can choose for placing a number of channels in parallel rather than placing all capture probe module in series. Different capture probe modules may comprise different capture probes thereby binding to different analytes. This means that in different capture probe modules different analytes may be detected.

Membranes which are suitable according to the present inventions may be the selected from the group comprising, negative and positive photo-resist materials e.g. a photosensitive epoxy resin known as SU-8.

Capture probes according to the present invention may be capable to bind to the targeted analytes specifically in a said fluid. Capture probes may be nucleic acids such as a DNA, RNA, aptamers, antibodies, Fab fragments, Fc tails. Capture probes may be proteins, such as e.g. receptors, antibodies. Antibodies may be used in form of polyclonal or/and monoclonal antibodies. A capture probe may be a drug or a cell or other chemical compounds.

Capture probes may be bound to the solid phase, the membrane, already before the test, for example by adsorptive or covalent interactions. A covalent interaction can be formed, as an example, by the reaction of an amine end-capped DNA string with the unreacted epoxy groups at the surface of the SU8 structures. Also other specific high affinity interactions may be used, e.g. streptavidin or avidin/biotin or antibody/antigen interactions. On the other hand, the solid phase capture probe can also be present in a form which is capable of binding to the solid phase, i.e. it is only bound to the solid phase during the assay and preferably by means of a high affinity interaction. As another preferred alternative the surface of the solid phase may be functionalized with specific chemical end-groups, for example by graft polymerization. In a specific example the surface is modified with N-hydroxysuccinimide groups which react with primary amino groups in many protein antibodies. Thus, the device can be tuned for any desired application.

The biomolecules of interest (i.e. analytes) bind specifically to the corresponding capture probes. Various detection methods have been developed to detect the bound analyte whether the specific binding has occurred. The detection method according to the present invention may be an optical detection method based on e.g. fluorescence; it may be based on refractive index changes, spectral changes, but also changes in localized magnetic or dielectric behavior. In principle, all marking techniques which can be used of the type described are employed, including marking with radio isotopes, enzymes, fluorescent, chemoluminescent or bioluminescent labels and directly optically detectable colour markers, such as, for example, gold atoms and dye particles, magnetic particles.

So-called sandwich assays in which two capture probes, e.g. two receptors or two antibodies, directed against different epitopes of the analytes to be determined, are frequently used for the quantitative determination of analytes in a sample. In this method a first soluble capture probe, e.g. receptor / antibody, is preferably directly or indirectly coupled with a signal- generating system i.e. with a label, whereas a second capture probe, e.g. receptor/antibody, is present coupled to a solid phase or is provided with a binding component such as e.g. biotin which is able to bind to a suitably coated solid phase.

In case of a sandwich immunoassay it is preferable to mark both antibodies with parts of a detection system which, when both antibodies are integrated into a single sandwich, permit signal generation or signal triggering. Such techniques can be designed in particular as fluorescence extinction detection methods.

Exemplary, a sandwich immunoassay may be conducted with a first antibody immobilized on the membrane. The analyte containing the analyte is incubated on the membrane. Excess analyte is removed by washing. A biotinylated second antibody is added. In order to remove unspecific binding another washing step is performed. Cy5/Alexa 647 labeled streptavidin is then added which will bind to the antibody-analyte-antibody complex and will give the signal which may be measured via a CCD camera.

Alternatively the immunoassay may be performed in a one-step assay with help of an antibody or another capture probe which is immobilized on a substrate, the membrane, whereas the analyte is labeled.

Alternatively, the immunoassay may be based on the known principle of competitive immunoassay, the most typical member of which in turn is a radioimmunoassay (RIA) and an enzyme immunoassay (Hartter et al.; Clin Chem Lab Med 2000, 38 (1) 27 -32). In the case of a competitive immunoassay both the sample containing the unlabeled analyte and a labeled analyte are incubated together on the solid substrate. The analyte may be labeled e.g. via biotinylation. After incubation unbound analytes are removed during a washing step. Strepatavidin-Cy5 solution is added. In this exemplified assay there is an inverse relationship between signal intensity and the amount of analyte present. Higher signal would mean that the analyte of interest is present in minute quantities. The signal intensity may be measured via a CCD camera.

Further immunoassay embodiments are known to the person skilled in the art and may be performed according to the present invention.

The device may further comprise a detection device. A detection device may be selected from the group consisting of a photodiode light sensor for measuring absorbtion or emission of light, CCD camera for measuring optical signals from an array of probes, confocal microscopy, GMR (giant magneto resistance) sensor - for magnetic particles, gamma detector (radio-isotopes), a capacitance bridge - for measuring changes in dielectric properties.

The channel design is such that the pressure drop over the capture probe module is relatively small, allowing the placement of a large number of such capture probe modules in series without the need of using large pressure to transport the analyte through the channel. Also, the design of the capture probe module comprising elements, is such that the average diffusion distance of target molecules to reach the capture probe at the surfaces of the membranes is relatively small, which translates into short assay times.

The present invention thus combines properties of flow-through and flow-over devices so that the specific advantages of both types of devices are provided.

Furthermore, a method for manufacturing a diagnostic device is provided and a method for detecting an analyte in a fluid using a device according to the present invention.

Thus, the subject of the present invention is a method for manufacturing a diagnostic device for detecting an analyte in a fluid in the device according to the present invention, comprising the steps of:
(i) applying a layer of photoresist on a substrate;
(ii) creating a latent image in the photo resist by for example exposing through a mask or using a holographic setup, wherein
   steps (i) and (ii), or solely step (ii) may be repeated;
(iii) when using a negative photo resists the unexposed areas may be dissolved by solvent development, thereby removing the non-exposed portions of the photoresist; when using a positive photoresists the exposed areas may be dissolved by solvent development.
(iv) applying a capture probe for binding said analyte to the carrier substrate.

In a preferred embodiment, subject of the present invention is a method, wherein the exposing step comprises:
(a) holographically exposing the photoresist to create a latent image of a plurality of elongate sub-micrometer sized lamellae structures which are the elements and
(b) exposing the photoresist by mask exposure to UV light to create a latent image of the channel
parallel to said structures.

A person skilled in the art will understand that there are further possibilities to manufacture the inventive device. Direct-write technologies as ion beam writing, electron beam lithography, replication techniques by molding of a liquid polymer precursor, etc.

Subject of the present invention is further a method for detecting an analyte in a fluid using a device according the present invention comprising the following steps:
applying fluid containing an analyte to the flow channel;
making the fluid flow through the flow channel;
and
detecting the captured analyte on the carrier substrate.

Subject of the present invention is further the above described method for detecting an analyte in a fluid using a device according to the present invention wherein the channels may be closed from their top side in order to avoid transport of analyte along unwanted voids left between the capture probe modules by adhering a cover plate covered with soft compliant material facing the channels and the capture probe modules.

In a preferred embodiment the compliant material is loosely crosslinked polydimethylsiloxan.

These and other aspects of the invention will be apparent from and elucidated with reference to the embodiments described hereinafter.

### BRIEF DESCRIPTION OF THE DRAWINGS

Figure 1 schematically shows the basic working principle of a diagnostic device together with an example of a fluorescence pattern obtained from a microarray device.
Figure 2 shows a membrane for use in a flow-through diagnostic device in different enlargements, wherein the membrane is available as Pamgene biosensor [www.pamgene.com].
Figure 3 schematically shows the channel structure of a diagnostic device according to one embodiment of the present invention together with a top-view scanning electron microscopy picture of a section comprising membranes as elements.
Figure 4 shows a scanning electron microscopy picture of several flow channels in a diagnostic device according to an embodiment of the present invention, the channels having alternating open sections and sections with membranes.

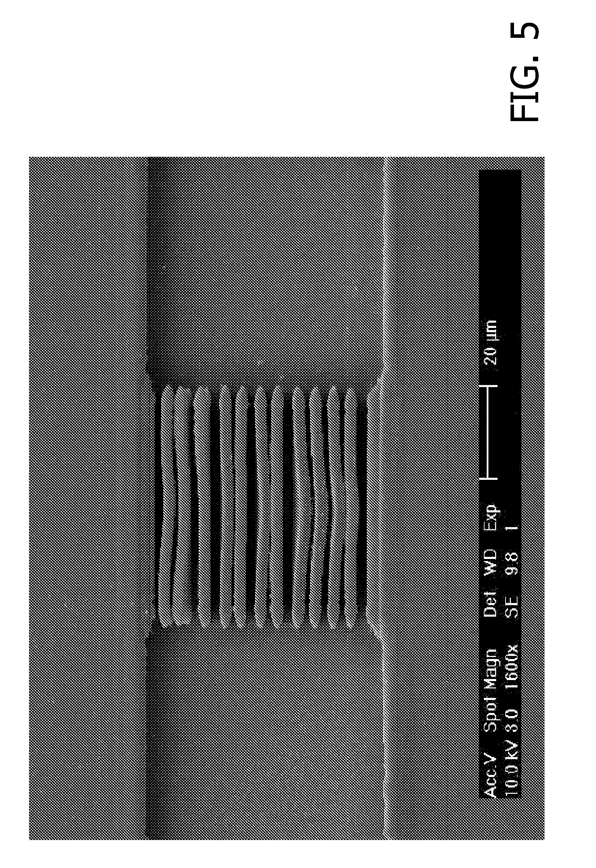

Figure 5 shows an exemplary preferred embodiment of the flow channel, where the flow channel width is 50 µm and the element width is 2 µm, the total length of the capture probe module being 50 µm.
Figure 6 shows the recovery performed on a Su8 coated glass slide.
Figure 7 shows the correlation between the number of droplets and the diameter. Note that this is performed on a flat sheet of Su8.

### DETAILED DESCRIPTION OF EMBODIMENTS

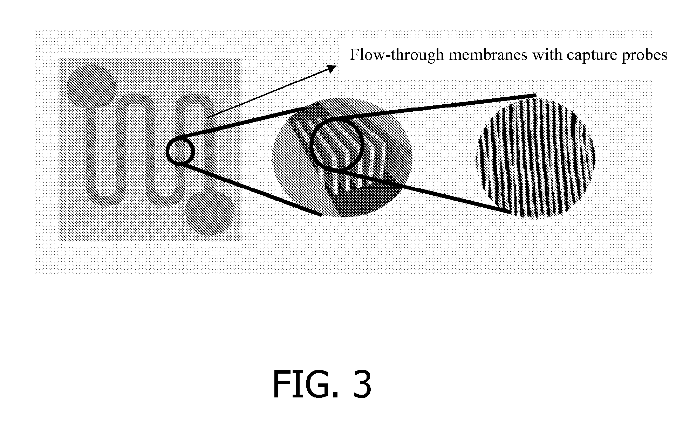

Figure 3 schematically shows the structure of a flow channel in a diagnostic device according to an embodiment of the present invention.

The fluid comprising the analyte to be detected enters the flow channel via an application zone which is illustrated by one of the circular areas at both ends of the flow channel in Figure 3(a). The fluid flows through the flow channel driven, for example, by capillary action, applied pressure difference, etc. Along the length of the flow channel, a plurality of sections, i.e. capture probe modules, is provided, each section comprising a plurality of microstructures, i.e. elements, on which the capture probes are arranged. In an embodiment of the invention, different capture probes are arranged in each of these sections or spots (capture probe module), in order to detect different analytes when the fluid flows through the flow channel. At the end of the flow channel, the fluid is extracted via an extraction zone, again illustrated by a circular spot.

Figure 3(b) schematically shows an enlarged view on one of the spots (capture probe module) present in the flow channel, Figure 3(c) is a top-view scanning electron microscopy picture of a portion of a spot (capture probe module). According to the embodiment shown in Figures 3(b) and 3(c), elements are arranged in the spot (capture probe module) in parallel with the channel and further in parallel to each other, respectively, separated by a predetermined distance. It is, however, noted that instead of providing a number of parallel elements in the form of plates, also other shapes and arrangements of the microstructure present in a particular spot may be chosen, such as interrupted plates, oblique plates, small pillars, horizontal pores, etc., as long as the arrangement in a membrane is such that the pressure drop over the capture probe module (spot) remains small enough to allow the placement of a large number of spots in series without the need of using large pressures to transport the analyte through the channel.

The elements disposed in a particular spot comprise capture probes such as oligonucleotids, nucleic acids, DNA, RNA, receptors, proteins or antibodies which are immobilized on the surface of the membranes. Conveniently, these probes can be applied by inkjet printing which is helped by the large capillary action of the closely packed elements. This also prevents contamination of the open channels with capture probes which would result in high background signals and a low signal-to-noise ratio. Apart from inkjet printing, various other methods can be chosen from simple pipetting by hand to complex offset printing.

The structure of the flow channel on the detection device may be such that the flow channel comprises straight sections which are connected via curved sections in order to result in a single flow channel through which a fluid may flow from an application zone to an extraction zone, as illustrated in Figure 3(a). However, it is also possible to arrange several channels in parallel through which a fluid may simultaneously flow, for example by branching the flow channels from a single channel connected with the application zone.

A flow channel preferably has a width of about 1000 µm or less, for example about 50 µm. Preferably, a series of sections or spots (capture probe module) comprising elements as porous membranes are arranged along the length of the flow channel. Each membrane extends across the whole width of the channel and has a length which preferably corresponds to the width of the channel, that is, 1000 µm or less. The different capture probe modules are separated from each other by portions of the flow channel which remain open. These open sections also may have a length corresponding to the length of the sections, so as to avoid any interference between the different capture probe modules.

In order to detect whether in the spots (capture probe module) the specific binding has occurred, the known methods may be used, such as optical methods based on fluorescence, refractive index changes, spectral changes, etc., but also changes in localized magnetic or dielectric behavior may be used.

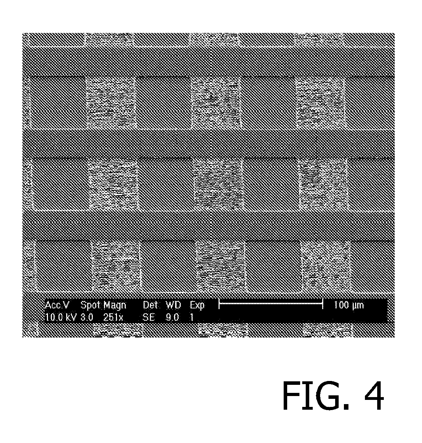

Figure 4 shows a scanning electron microscopy picture of several flow channels in a diagnostic device according to an embodiment of the present invention. In the channels, open sections and sections with membranes (capture probe modules) are alternately arranged. The parallel microchannels shown in Figure 4 are connected to each other by curved sections (not shown) at their respective ends, so that they form one continuous microchannel.

The present invention also relates to a manufacturing method of the diagnostic device. Conveniently, this is done in a negatively responding photosensitive layer in which the channels are made by a lithographic procedure, that is, the areas exposed during the mask irradiation remain and the unexposed areas can be dissolved by solvent development. According to an embodiment of the method according to the present invention, the membranes are made in the same layer of photosensitive material by holographic exposure through a second mask before the development of the first exposure.

For example, a specific flow-through channel structure may be produced by applying a 50 µm thick layer of negative photoresist material, for example SU8 (commercially available from MicroChem Corporation) on a glass substrate). During a pre-bake step, the photoresist is exposed to a temperature of 65°C for two minutes followed by ten minutes at 95°C - however, times adapted to layer thicknesses. This pre-bake step insures that all solvent has evaporated and that the photoresist is in a glassy state, enabling the use of multiple exposure steps. The sample is then holographically exposed to create a latent image of sub-micrometer lamellae with light of 351 nm at 140 mJ/cm². Subsequently, the channels are recorded in the photo resistant material by a mask exposure to UV light for 30 seconds at 200 mW/cm².

After the exposures, the sample is heated to 65°C for one minute and to 95°C for two minutes to induce cross-linking of the photoresist. The non-crosslinked areas are washed away with a SU8-developer (mr-Dev600, commercially available from MicroChem Corporation). The sample is then rinsed with propanol-2. In case the elements to be formed on the substrate have a diameter in the micrometer range or larger, a single mask exposure step can replace the two-step exposure described above, limiting the feature sizes to the mask design.

The method of the invention can be used in the production of devices for the efficient and sensitive scanning of fluids on the present and/or amount of DNA, proteins, antibodies, tissue, cells, drugs and other chemical compounds. By functionalizing the surface with specific chemical end-groups, for example by graft polymerization, the device can be tuned for any desired application. In addition to minimize a specific binding, LIFE sensor can be blocked before performing the immunoassay e.g. 5%BSA, gelatin, 5% Casein

In other to close the channels, containing the elements coated with capture probes, a flat cover is used. This cover can be adhered to the bottom plate by an adhesive. The cover plate may contain openings through which the sample can be added via tubings. In a preferred embodiment the cover is coated with a soft compliant material such that surface unevenness is cushioned. Also small height difference in the plate elements are rep[aired avoiding flow of analyte along voids. A suited compliant material is polydimethyl siloxane (PDMS: Sylgard 184 - Dow Chemical) which is coated as a 50 µm layer on the cover plate with the PDMS layer facing the channels and the capture probe modules. For some read-out principles the cover plate must be transparent and a PDMS coated polymethyl methacrylate provides a suited and robust cover.

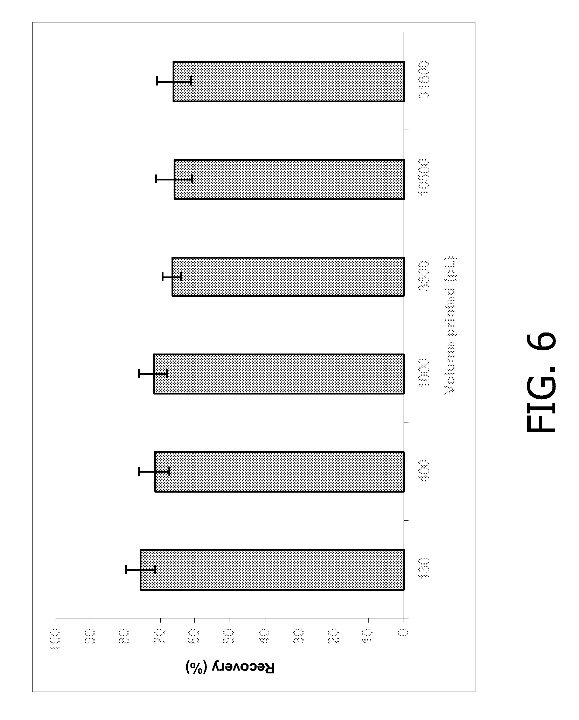

Figure 6 shows the number of molecules still present after a blocking step and a washing step. The blocking step comprises of adding 5% BSA to the substrate and incubated it for 60 minutes at room temperature. Then the substrate is washed 3 times for 5 minutes with 1xPBS + 0.05% Tween-20, to remove all unbound material. The recovery, so the number of molecules bound to the membrane is calculated by dividing the intensity after blocking and washing with the intensity before blocking washing.

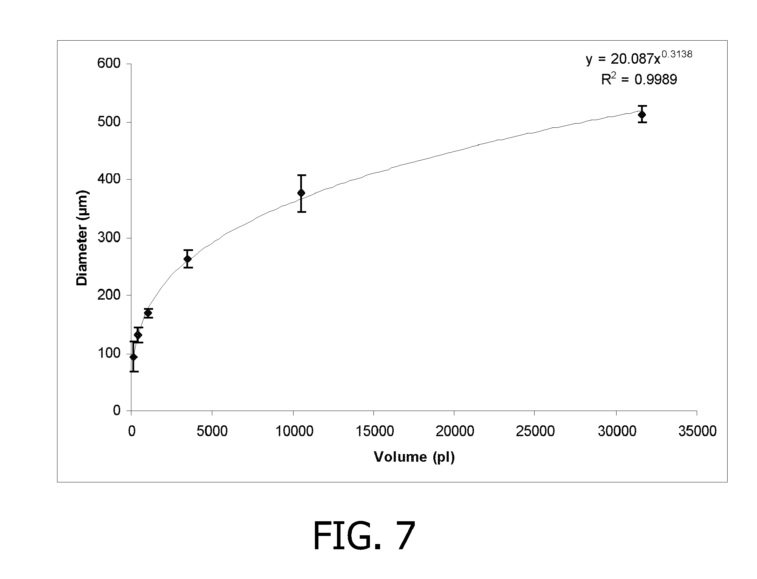

Figure 7 shows the correlation between the diameter and the volume printed. It is shown that the high volume can be printed on the surface on a relative small substrate surface. This allows us to better control the printing and the depositing in the life elements.

## Claims

1. Diagnostic device for detecting an analyte in a fluid, comprising
a capture probe module, wherein the capture probe module comprises,
(i) a flow channel comprising microchannels, and
(ii) a plurality of elements
comprising at least one capture probe or alternatively being capable of immobilizing at least one capture probe for detecting said analyte, wherein said elements are arranged in said flow channel in such an order that adjacent elements form part of said microchannels.

2. Diagnostic device according to claim 1, wherein the element is a porous membrane.

3. Device according to claim 1 to 2, wherein the elements are arranged in parallel with the flow channel.

4. Device according to claim 1 to 3, wherein the elements have a thickness of about 5 µm or less.

5. Device according to claim 1 to 4, wherein the elements have a thickness of about 2 µm and/or are separated by about 2 µm in the capture probe module.

6. Device according to claim 1 to 5, wherein the elements have an elongated, plate-like form.

7. Device according to claim 1 to 6, wherein the elements are arranged in parallel to each other within said capture probe module.

8. Device according to claim 1 to 7, wherein the elements have the form of pillars extending perpendicularly through the flow channel.

9. Device according to claim 1 to 8, wherein said flow channel has a width of 100 µm or less.

10. Device according to claim 1 to 9, wherein said capture probe module has a length of 100 µm or less.

11. Device according to claim 1 to 10, wherein the flow channel comprises in series several capture probe modules.

12. Device according to claim 11, wherein elements in different capture probe modules comprise different capture probes.

13. Device according to claim 11 to 12, wherein capture probe modules are separated by at a distance corresponding at least to the length of each capture probe module.

14. Device according to claim 1 to 13, comprising several flow channels in parallel.

15. Device according to claim 1 to 14, further comprising a detection device.

16. Device according to claim 1 to 15, wherein said capture probe is selected from a group comprising nucleic acids, a protein, an antibody, a tissue, a cell, and a drug.

17. Method for manufacturing a diagnostic device for detecting an analyte in a fluid in the device according to claim 1, comprising the steps of:
(i) applying a layer of negative photoresist on a substrate;
(ii) exposing the negative photoresist through a mask to irradiation wherein exposed areas remain after masked irradiation, wherein
steps (i) and (ii) or solely step (ii) may be repeated;
(iii) unexposed areas are dissolved by solvent development,
thereby removing the non-exposed portions of the negative photoresist;
(iv) applying a capture probe for detecting said analyte to the carrier substrate.

18. Method according to claim 17, wherein a positive photoresist is used.

19. Method according to claim 17-18, wherein the exposing step (ii) comprises
(a) holographically exposing the photoresist to create a latent image of a plurality of elongate sub-micrometer sized lamellae structures and
(b) exposing the photoresist by mask exposure to UV light to create a latent image of the channel parallel to said structures.

20. Method for detecting an analyte in a fluid using a device according to claim 1 comprising the following steps:
(i) applying fluid containing an analyte to the flow channel;
(ii) letting the fluid pass through the flow channel either by capillary filling or applied pressure difference
and
(iii) detecting the captured analyte on the carrier substrate.
